# EUROPÄISCHE PATENTSCHRIFT

(11) **EP 1 200 408 B1**
(45) Veröffentlichungstag und Bekanntmachung des Hinweises auf die Patenterteilung: **03.09.2003**
(21) Anmeldenummer: 00958184.4
(22) Anmeldetag: 27.07.2000
(51) Int. Cl.: C07D 215/38, C23C 22/00, C09D 5/00

(54) **HAFTVERMITTLER**
BONDING AGENT
AGENT ADHESIF

(30) Priorität: 03.08.1999 DE 19936472
(43) Veröffentlichungstag der Anmeldung: 02.05.2002
(73) Patentinhaber: STN ATLAS Elektronik GmbH, 28305 Bremen (DE)
(72) Erfinder: HÜLSEBUSCH, Marlies, 33613 Bielefeld (DE); LINDNER, Jürgen, D-27777 Ganderkesee (DE)
(74) Vertreter: Winkler, Andreas, Dr.
(86) Internationale Anmeldenummer: DE0002481
(87) Internationale Veröffentlichungsnummer: WO01009099

(56) Entgegenhaltungen:
- EP-A- 0 413 216
- DD-A- 301 777
- GB-A- 794 059
- GB-A- 966 488
- US-A- 4 500 601
- CHEMICAL ABSTRACTS, vol. 129, no. 7, 17. August 1998 (1998-08-17) Columbus, Ohio, US; abstract no. 89578x, HUANG, YINGPING ET AL.: "Synthesis of a new reagent 2,3,7-trihydroxy-9-(3,5-dibromo-4-(8-hydro xy-5-quinolylazo)) and study on the spectrophotometric determination of tungsten." XP002160464 -& DATABASE CHEMICAL ABSTRACTS [Online] 129:89578, XP002160475 & HUAXUE SHIJI, Bd. 20, Nr. 2, - 1998 Seiten 68-71,
- CHEMICAL ABSTRACTS, vol. 128, no. 9, 2. März 1998 (1998-03-02) Columbus, Ohio, US; abstract no. 102752a, RAY, A. ET AL.: "Construction of template ligand with 8-hydroxyquinoline" XP002160465 -& DATABASE CHEMICAL ABSTRACTS [Online] CA 128:102752, XP002160476 & J. APPL. POLYM. SCI., Bd. 67, Nr. 7, - 1998 Seiten 1215-1219,
- CHEMICAL ABSTRACTS, vol. 126, no. 18, 5. Mai 1997 (1997-05-05) Columbus, Ohio, US; abstract no. 245971r, AIELLO, IOLINDA ET AL.: "Monomeric and polymeric oxovanadium(IV)complexes containing 5-(4'-alkyl-phenylazo)-8-hydroxy-quinoline ligands." XP002160466 -& DATABASE CHEMICAL ABSTRACTS [Online] CA 126:245971, XP002160477 & INORG. CHIM. ACTA, Bd. 255, Nr. 1, - 1997 Seiten 133-137,
- CHEMICAL ABSTRACTS, vol. 116, no. 20, 18. Mai 1992 (1992-05-18) Columbus, Ohio, US; abstract no. 207088, LI, KEAN ET AL.: "Study on enrichment and separation of trace elements with silica gels modified by heterocyclic azo dyes." XP002160467 -& DATABASE CHEMICAL ABSTRACTS [Online] CA 116:207088, XP002160478 & CHIN. CHEM. LETT., Bd. 2, Nr. 8, - 1991 Seiten 633-636,
- CHEMICAL ABSTRACTS, vol. 51, no. 1, 10. Januar 1957 (1957-01-10) Columbus, Ohio, US; abstract no. 412b, EIJI OCHIAI ET AL.: "Syntheses of 4,6-diaminoquinoline derivatives. I." XP002160468 -& DATABASE CHEMICAL ABSTRACTS [Online] CA 51:412, XP002160479 & J. PHARM. SOC. JAPAN, Bd. 76, - 1956 Seiten 531-534,
- CHEMICAL ABSTRACTS, vol. 59, no. 6, 16. September 1963 (1963-09-16) Columbus, Ohio, US; abstract no. 6852e, L.P. KULEV ET AL.: "Azo derivatives of aminosalicylic acids and their complex inner salts." XP002160469 -& DATABASE CHEMICAL ABSTRACTS [Online] CA 59:6852, XP002160480 & IZV. TOMSK. POLITEKHN. INST., Bd. 102, - 1050 Seiten 29-32,
- CHEMICAL ABSTRACTS, vol. 94, no. 3, 19. Januar 1981 (1981-01-19) Columbus, Ohio, US; abstract no. 14922e, KHATER, M. M. ET AL.: "Effect of substituents on the ionization constants of some 8-quinolinol azo compounds." XP002160470 -& DATABASE CHEMICAL ABSTRACTS [Online] CA 94:14922, XP002160481 & J. PRAKT. CHEM., Bd. 322, Nr. 3, - 1980 Seiten 470-474,
- CHEMICAL ABSTRACTS, vol. 89, no. 19, 6. November 1978 (1978-11-06) Columbus, Ohio, US; abstract no. 163473h, ROUSHDI, I. M. ET AL.: "Arylazo heterocycles. Part II. Synthesis of arylazo quinoline ( and 2-isoxazolin-5-one derivatives) of possible antimicrobial activity." XP002160471 -& DATABASE CHEMCAL ABSTRACTS [Online] CA 89:163473, XP002160482 & EGYPT. J. PHARM. SCI. , Bd. 16, Nr. 4, - 1975 Seiten 415-420,
- CHEMICAL ABSTRACTS, vol. 78, no. 12, 26. März 1973 (1973-03-26) Columbus, Ohio, US; abstract no. 73631b, NAVRATILOVA, HANA ET AL.: "Monoazo dyes" XP002160472 & CS 143 866 A 15. November 1971 (1971-11-15) -& DATABASE CHEMICAL ABSTRACTS [Online] CA 78:73631, XP002160483
- CHEMICAL ABSTRACTS, vol. 109, no. 18, 31. Oktober 1988 (1988-10-31) Columbus, Ohio, US; abstract no. 162247g, AWAD I. M. A. ET AL.: "Synthesis of some 5-azo(4'-substituted benzenesulfamoyl)-8-hydroxyquinolines with antidotal and antibacterial activities." XP002160473 & J. INORG. BIOCHEM., Bd. 32, Nr. 2, - 1988 Seiten 77-89, -& DATABASE CHEMICAL ABSTRACTS [Online] CA 109:162247, XP002160484
- CHEMICAL ABSTRACTS, vol. 96, no. 23, 7. Juni 1982 (1982-06-07) Columbus, Ohio, US; abstract no. 198702d, CI, YUNXIANG ET AL.: "Some advances in studying the relation between structure and reactivity of organic reagents - effects of substituents" XP002160474 & HUAUE SHIJI, Bd. 1, - 1982 Seiten 33-39, -& DATABASE CHEMICAL ABSTRACTS [Online] CA 96:198702, XP002160485

## Beschreibung

Die vorliegende Erfindung bezieht sich auf die Verwendung von 8-Hydroxychinolinderivaten als Haftvermittler zwischen metallischen Oberflächen und organischen Polymerschichten. Als metallische Oberflächen kommen insbesondere Eisenlegierungen und mit Silber oder Gold beschichtete Keramikoberflächen, z.B. Piezokeramiken, in Betracht. Als organische Polymerschicht kommen insbesondere Polyurethane in Betracht, z.B. Klebstoffe und Lacke. Die organische Polymerschicht kann bei Verwendung des erfindungsgemäßen Haftvermittlers aus einer organischen Lösung oder einer wäßrigen Dispersion der Lack- oder Klebstoffbestandteile aufgebracht werden, die erst auf der Haftvermittlerschicht vollständig auspolymerisieren.

Bisher verwendete Haftvermittler, auch Primer genannt, wie z.B. Zink-Chromat-Primer, sind aufgrund ihres Schwermetallgehaltes toxisch und daher auch für die Umwelt schädlich. Die Aufgabe besteht vorliegend darin, einen toxikologisch unbedenklichen und umweltverträglichen Haftvermittler bereitzustellen, der organische Polymerschichten wie z.B. Lack- und Klebstoffschichten fest mit metallischen Oberflächen verbinden kann. Weiterhin stellt sich vorliegend die Aufgabe, eine Verfahren zur Herstellung geeigneter Haftvermittler aufzufinden, das in der Lage ist, mit wenigen Reaktionsschritten eine ausreichend hohe Reaktionsausbeute zu ergeben, so daß keine umfangreichen Aufreinigungs- oder Isolierungsschritte zur Gewinnung des Haftvermittlers aus der Reaktionsmischung erforderlich sind.

Die US 4 500 601 A offenbart die Verwendung von 8-Hydroxychinolinderivaten als Haftvermittler auf metallischen Oberflächen, die reaktive Gruppen tragen, mittels der sie mit anderen Monomeren copolymerisiert sind. Derartige Copolymere können ohne weitere zusätzliche Komponenten eine Beschichtung auf metallischen Oberflächen bilden.

Die DD 301 777 A9 offenbart die Herstellung eines Ionentauschers (Beispiel 7), durch Umsetzung von Zinkoxid mit zwei 8-Hydroxychinolinderivaten, die an einer azogekoppelten Ethylphenylgruppe Säuregruppen tragen, nämlich eine Carboxygruppe bzw. eine Diphosponsäuremonohydroxylmethylgruppe. Die freien Säuregruppen dienen als Ionentauschergruppe.

Die erfindungsgemäße Lösung wird durch die Modifizierung von 8-Hydroxychinolin mit organischen Diazoniumverbindungen bereitgestellt. Es wurde gefunden, daß 8-Hydroxychinolinderivate dann besonders als Haftvermittler geeignet sind, wenn sie einen Substituenten tragen, der über eine Azokupplung verbunden ist und der eine Gruppe trägt, die zur Reaktion mit Komponenten der aufzubringenden organischen Polymerschicht fähig ist.

Ein Haftvermittler auf Basis von 8-Hydroxychinolinderivaten ist als organisches Produkt prinzipiell chemisch oder biologisch zu unschädlichen Produkten abbaubar.

8-Hydroxychinolin, auch bekannt als 8-Chinolinöl oder Oxin, ist in der Medizin als Fungizid und Antiseptikum, in der Analytik als organischer Komplexbildner für mehr als 40 verschiedene Metalle bekannt.

Die erfindungsgemäßen Haftvermittler zeigen eine sehr hohe Feuchtigkeitsbeständigkeit und gute Korrosionsschutzeigenschaften im Zusammenhang mit dem Polymerverbund. Diese Haftvermittler weisen überdies eine nur geringe Toxizität auf. Für den Auftrag der Haftvermittler auf die zu beschichtenden Oberflächen wird nur ein geringer Temperatureinfluß auf die Unterlage ausgeübt und überdies eine hohe Konstanz bzw. Reproduzierbarkeit des Auftrages erzielt.

Die Azokupplung eines organischen Substituenten, der eine polymerspezifische Gruppe trägt, an das 8-Hydroxychinolin kann mittels der entsprechenden Diazoniumverbindung des Substituenten erzielt werden. Die Diazoniumverbindung des organischen Substituenten, der eine weitere reaktive polymerspezifische Gruppe trägt, läßt sich durch Umsetzen der entsprechenden Aminverbindung mit Nitrit, z.B. Natriumnitrit, erreichen.

Die mindestens eine zur Polymerisation mit Komponenten organischer Polymere fähige Gruppe ist bevorzugt eine freie Amino-, Carboxy-, Epoxy-, Hydroxy- oder Alkenylgruppe.

Bevorzugt kann auch eine Mischung von 8-Hydroxychinolinderivaten verwendet werden.

Erfindungsgemäß ist auch eine metallische Oberfläche, die durch eine erfindungsgemäße Verwendung eines 8-Hydroxychinolinderivats als Haftvermittler beschichtet ist. Dabei ist der Haftvermittler bevorzugt mit Komponenten organischer Polymere polymerisiert, und die metallische Oberfläche kann bevorzugt eine Eisenlegierung oder eine mit Silber oder Gold beschichtete Keramikoberfläche sein.

Das allgemeine Reaktionsschema für die Azokupplung des Substituenten, der eine reaktive polymerspezifische Gruppe trägt, an das 8-Hydroxychinolin erfolgt nach dem allgemeinen Reaktionsschema: 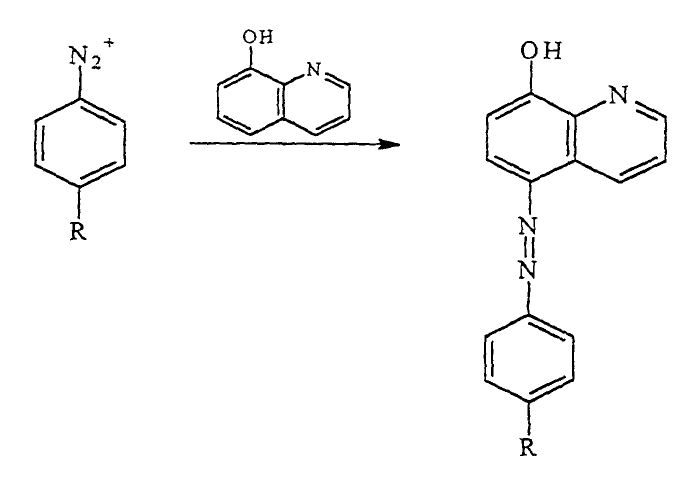 R = reaktive Gruppe, insbesondere Amino-, Carboxy-, Epoxy- oder Vinylgruppe.

Anstelle des Benzolringes im Substituenten lassen sich auch andere organische Reste verwenden, die als Spacermolekül fungieren. Im hier gezeigten allgemeinen Reaktionsschema dient der Benzolring gleichzeitig als Spacer. Als Spacer können auch Alkenylreste mit 2 bis 6 Kohlenstoffatomen dienen, wie Ethenyl, Propenyl, Butenyl, Pentenyl und Hexenyl.

Es können auch Substituenten über eine Azokupplung mit dem 8-Hydroxychinolin verbunden werden, die mehr als nur eine reaktive Gruppe aufweisen.

Die Azokupplung des Diazoniumsubstituenten erfolgt fast ausschließlich in 5-Stellung des 8-Hydroxychinolins.

Die vorliegende Erfindung wird anhand der nachfolgenden Beispiele näher erläutert, die jedoch nicht als Beschränkung aufzufassen sind.

Kurze Beschreibung der Figuren:
Figur 1 zeigt das FT-IR-Drift-Spektrum eines Haftvermittlers auf Basis von 8-Hydroxychinolin.
Figur 2 zeigt das FT-IR-Spektrum des Haftvermittlers, auf Silberblech aufgetragen.
Figur 3 zeigt das FT-IR-Drift-Spektrum eines Haftvermittlers auf Basis von 8-Hydroxychinolin.
Figur 4 zeigt das FT-IR-Spektrum des Haftvermittlers nach Auftragen auf ein vergoldetes Edelstahlblech.
Figuren 5 und 6 zeigen den Aufbau einer Beschichtung auf metallischen Oberflächen 1,5 aus Haftvermittler 2,4 und organischem Polymer 3.

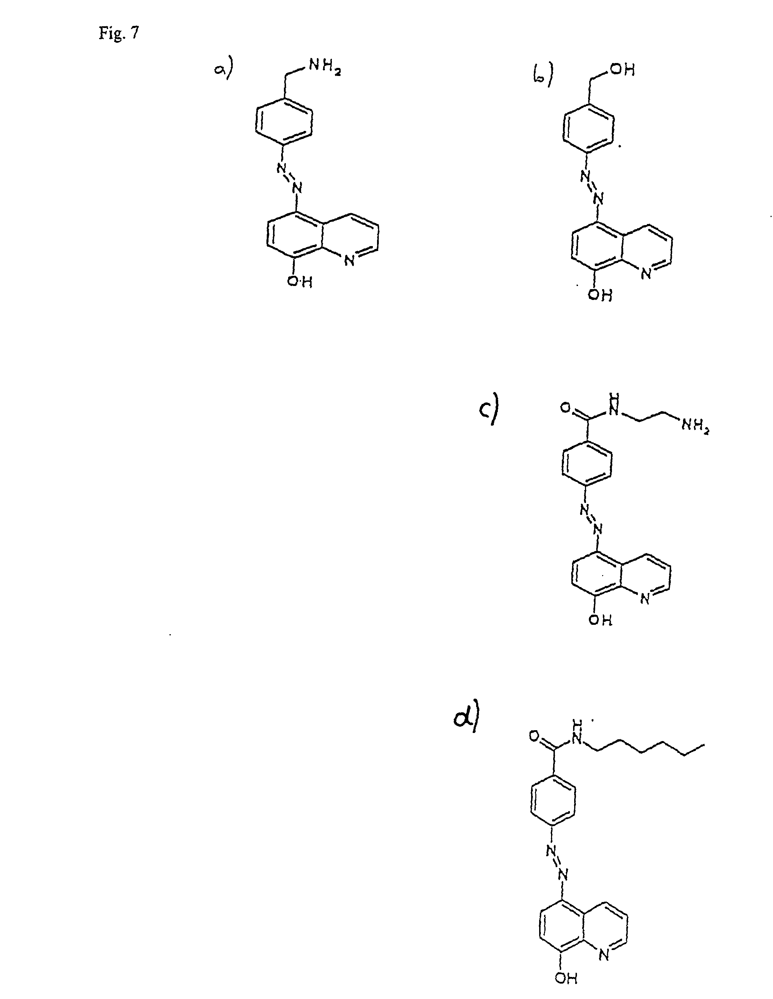

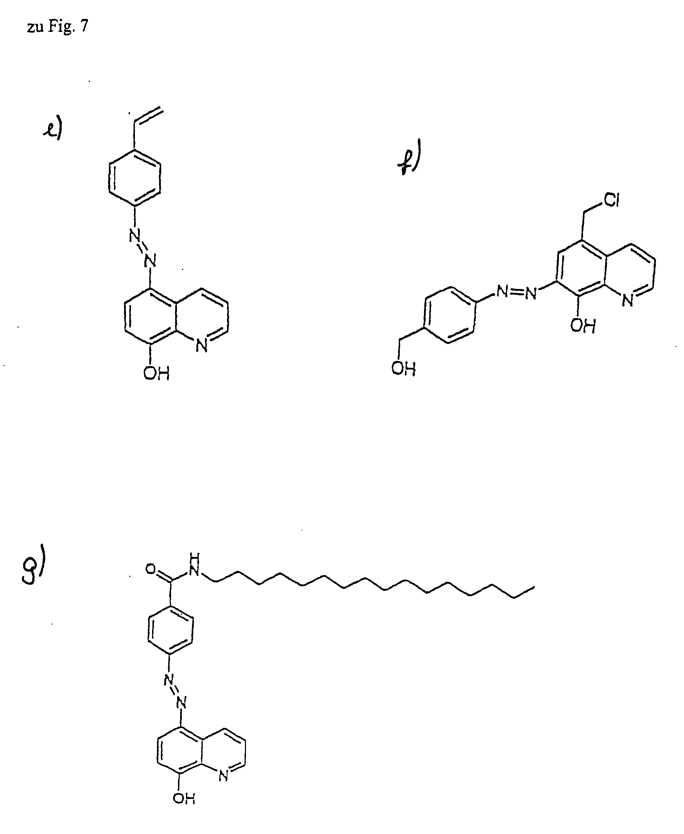

Figur 7 zeigt Strukturformeln erfindungsgemäßer Haftvermittler.

### Beispiel 1: Synthese von 5-(p-Aminobenzylazo)-8-hydroxychinolin

2,44 g (0,02 Mol) p-Aminobenzylamin werden in eine Mischung aus 9 ml (0,1 Mol) einer 37%igen Salzsäure und 35 ml Wasser gegeben. Die Lösung wird auf 0-5°C gekühlt. Die Diazotierung wird durch Zugabe einer Lösung aus 1,38 g (0,02 Mol) NaNO₂ in 10 ml Wasser gestartet. Anschließend wird für 30 Minuten bei Raumtemperatur gerührt. Diese Lösung aus diazotiertem p-Aminobenzylamin wird langsam zu einer kalten Lösung (0-5°C) aus 2,90 g 8-Hydroxychinolin in 80%iger Essigsäurelösung getropft, so daß die Temperatur nicht über 5°C ansteigt. Nach vollständiger Zugabe wird für weitere 2 Stunden bei dieser Temperatur gerührt, dann läßt man langsam auf Raumtemperatur erwärmen. Dabei fällt bereits ein Teil des Produktes aus. Die vollständige Fällung wird durch vorsichtiges Neutralisieren unter Kühlung mit wäßriger Ammoniaklösung erreicht. Das ausgefällte Produkt wird abgenutscht und mit Eiswasser gewaschen.

Die Zersetzungstemperatur beträgt 273°C. Das IR-Spektrum zeigt folgende Peaks: 3005, 2865, 1630, 1605, 1550, 1505, 1400, 1375, 1350, 1300, 1235, 1080, 850 cm⁻¹, ¹H-NMR-Spektrum (250 MHz, DMSO, Tieffeldverschiebung gegen TMS): 9,33 ppm (1 H), 8,99 ppm (1 H), 8.03 ppm (1 H), 8,02 ppm (2 H), 7,77 ppm ( 1 H ), 7,71 ppm (2 H), 7,25 ppm (1 H), 4.14 ppm (2 H).

### Beispiel 2: Synthese von 5-(p-Hydroxybenzylazo)-8-hydroxychinolin

2,46 g (0,02 Mol) 4-Aminobenzylalkohol werden in einer Mischung aus 5 ml konzentrierter HCI und 100 ml Wasser gelöst, auf 0-5°C gekühlt und mit 1,38 g (0,02 Mol) NaNO₂ in 10 ml Wasser diazotiert. 2,90 g (0,02 Mol) 8-Hydroxychinolin werden in 10 ml 1 M NaOH gelöst und auf 0-5°C gekühlt. Dieser Lösung wird langsam die Lösung des diazotierten 4-Aminobenzylalkohols zugegeben. Es wird für weitere 16 Stunden bei dieser Temperatur rühren gelassen. Anschließend wird mit konzentrierter Essigsäure neutralisiert und das enstandenen Produkt abgenutscht.

Der Schmelzpunkt beträgt 221°C. Das IR-Spektrum zeigt folgende Peaks: 3290, 3065, 2920, 2865, 1570, 1475, 1280, 1225, 1190, 1020, 845, 790, 710 cm⁻¹. ¹H-NMR-Spektrum (250 MHz, DMSO, Tieffeldverschiebung gegen TMS): 9,31 ppm (1 H), 9,00 ppm (1 H), 8,01 ppm (1 H), 7,97 ppm (2 H), 7,76 ppm (1 H), 7,56 ppm (2 H), 7,26 ppm (1 H).

¹³C-NMR-Spektrum (62.9 MHz. Tieffeldverschiebung gegen TMS): 157.3 ppm, 151,5 ppm. 148,9 ppm, 145,5 ppm, 138,6 ppm, 137,9 ppm, 131,7 ppm, 127,4 ppm, 127,1 ppm, 123,0 ppm, 122,3 ppm, 114,6 ppm, 111,6 ppm, 62,5 ppm.

### Beispiel 3: Synthese von 5-(p-Vinylbenzylazo)-8-hydroxychinolin

2,98 g (0,025 Mol) p-Vinylanilin werden in 5 ml konzentrierter HCl in 100 ml Wasser gelöst. Die Lösung wird auf 0-5°C gekühlt und mit 1.73 g (0,025 Mol) NaNO₂ in 12 ml Wasser diazotiert. Diese Diazoniumlösung wird langsam zu einer kalten Lösung von 3,7 g (0,025 Mol) 8-Hydroxychinolin in 50 ml 2-Ethoxyethanol und einer Lösung aus 1 g NaOH sowie 5 g Na₂CO₃ in 50 ml Wasser gegeben. Die Lösung wird anschließend für 24 Stunden bei 0-5°C gerührt. Der ausgefallene Feststoff wird abgenutscht und mit Eiswasser gewaschen. Der Feststoff wird in Methanol umkristallisiert.

Der Schmelzpunkt liegt bei >300°C. IR-Spektrum: 3350, 3190, 3030, 2920 1740, 1690, 1600, 1580, 1410, 1340, 1270, 1165, 940, 790, 670 cm⁻¹. ¹H-NMR-Spektrum (250 MHz, DMSO, Tieffeldverschiebung gegen TMS): 9,33 ppm (1 H), 9,00 ppm (1 H), 8,02 ppm (2 H), 7,92 ppm (1 H), 7,75 ppm (1 H), 7,70 ppm (2 H), 7,23 ppm (1 H), 6,86 ppm (1 H), 6,00 ppm (1 H), 5,40 ppm (1 H).

Die erfindungsgemäßen Haftvermittler können ethanolischer Lösung auf eine metallische Oberfläche aufgebracht werden. Die Komplexierung der metallischen Oberfläche durch erfindungsgemäßer Haftvermittler auf Basis von 8-Hydroxychinolin wird in den folgenden Beispielen deutlich:

### Beispiel 4: Beschichtung einer Silberoberfläche mit Haftvermittler auf der Basis von 8-Hydroxychinolin

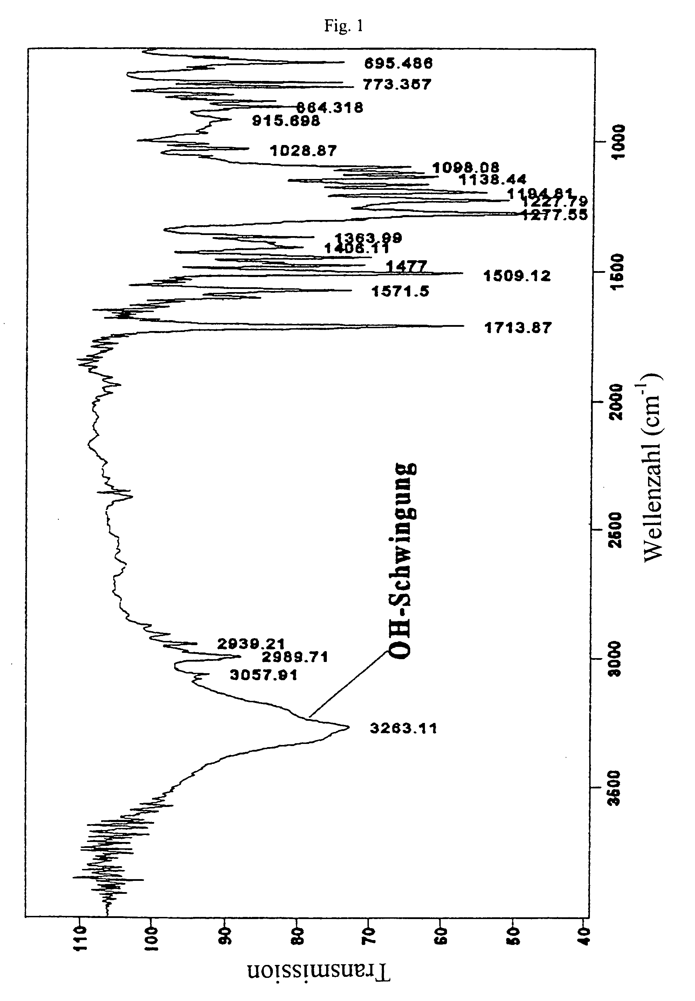

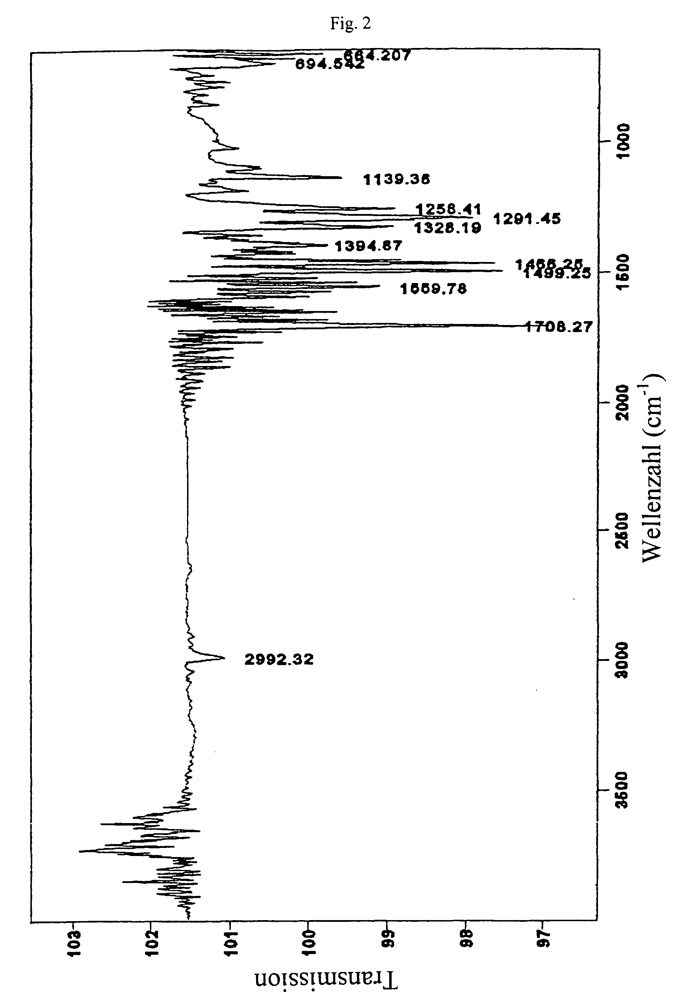

Der Vergleich der IR-Spektren eines Haftvermittlers auf Basis von 8-Hydroxychinolin. in Figur 1 dargestellt. mit dem auf Silberblech aufgetragenen Haftvermittler, in Figur 2 dargestellt, zeigt. daß die OH-Schwingung des Haftvermittlers nicht mehr auftritt, wenn er auf die Silberoberfläche aufgetragen ist.

### Beispiel 5: Beschichtung einer Goldoberfläche mit einem Haftvermittler auf Basis von 8-Hydroxychinolin

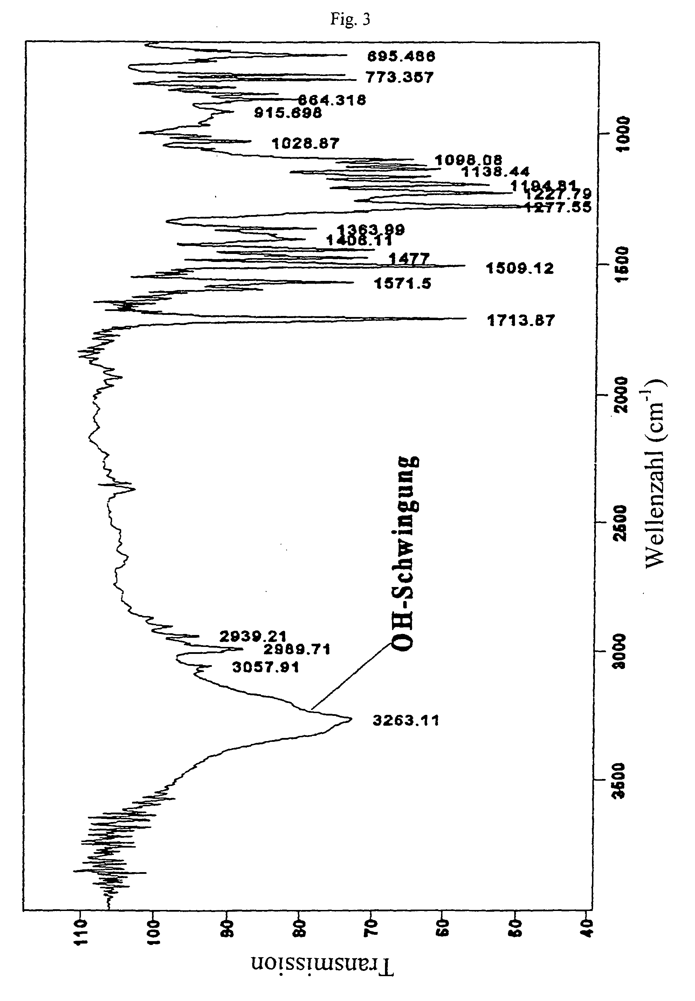

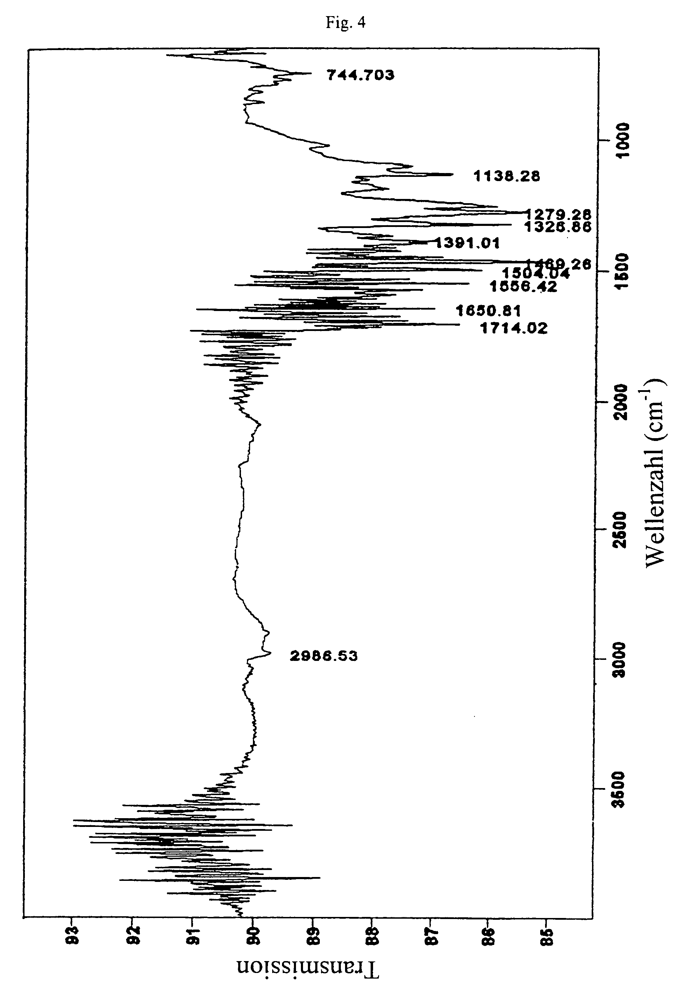

Der Vergleich der IR-Spektren eines erfindungsgemäßen Haftvermittlers, in Figur 3 dargestellt, und des auf vergoldetes Edelstahlblech aufgetragenen Haftvermittlers, in Figur 4 zeigt, daß die OH-Schwingung des Haftvermittlers nach Auftragen auf die Goldoberfläche nicht mehr vorliegt. Das Fehlen dieser OH-Schwingung belegt die Komplexbildung des erfindungsgemäßen Haftvermittlers mit der Goldoberfläche.

### Beispiel 6: Zugfestigkeitsprobe auf CrNi1810

Für die Zugfestigkeitsprobe nach DIN 53281 wurde CrNi1810 mit 25 mg 5-p-(Aminobenzylazo)-8-hydroxychinolin, gelöst in 250 ml Ethanol, durch Tauchen während 12 Stunden beschichtet. Anschließend wurde Epoxidklebstoff, Typ 9323 der Firma 3M aufgebracht und die Zugverfestigkeit nach Aushärtung und Konditionierung der Klebung gemessen. Bei Raumtemperatur ergab sich ein Wert von 19 N/mm², nach einer Auslagerung von 16 Stunden in Wasser bei über 95°C eine Wert von 19 N/mm², wiederum bei Raumtemperatur gemessen.

### Beispiel 7: Zugscherfestigkeitsprobe auf vergoldetem Messingblech

Vergoldetes Messingblech wurde mit 25 mg 5-(p-Vinylbenzylazo)-8-hydroxychinolin, in 250 ml Ethanol gelöst, im Tauchbad über 24 Stunden beschichtet. Anschließend wurde silbergefüllter Epoxidklebstoff aufgetragen und die Zugscherfestigkeit nach Aushärtung und Konditionierung der Klebung bei Raumtemperatur gemäß DIN EN1465 zu 9,9 N/mm² bestimmt.

### Beispiel 8: Zugscherfestigkeitsprobe auf vergoldetem Messingblech

Vergoldetes Messingblech wurde mit 5 mg 5-(p-Aminobenzylazo)-8-hydroxychinolin, in 200 ml Ethanol gelöst, durch Tauchen für 24 Stunden beschichtet. Anschließend wurde silbergefüllter Epoxidklebstoff aufgetragen und die Zugverfestigkeit nach Auswertung und Konditionierung der Klebung gemäß DIN EN 1465 bei Raumtemperatur zu 9.5 N/mm² bestimmt.

### Beispiel 9: Zugfestigkeitsprobe auf AlMg₃

AlMg₃ wurde mit 20 mg 5-(p-Vinylbenzylazo)-8-hydroxychinolin, in 250 ml Ethanol gelöst, durch Tauchen während 23 Stunden beschichtet. Anschließend wurde Polyurethan-Gießharz, z.B. Biresin 1305 aufgetragen und die Zugfestigkeit nach Auswertung und Konditionierung der Klebung gemessen, indem die Zugfestigkeit zweier mit den Stirnflächen aufeinander geklebter Rundproben mit 20 mm Durchmesser bestimmt wurde. Die Prüfung bei Raumtemperatur ergab eine Zugfestigkeit von 8 N/mm², nach Auslagerung in Wasser bei einer Temperatur oberhalb 95°C während 16 Stunden einen Wert von 4,3 N/mm², wobei wiederum bei Raumtemperatur gemessen wurde.

### Beispiel 10: Zugfestigkeitsprobe auf Edelstahl-Werkstoff Nr. 1.4571

Edelstahl Nr. 1.4571 wurde mit 20 mg 5-(p-Vinylbenzylazo)-8-hydroxychinolin, in 250 ml Ethanol gelöst, durch Tauchen während 23 Stunden beschichtet. Anschließend wurde Polyurethan-Gießharz, z.B. Biresin U1305 aufgetragen und die Zugfestigkeit wie in Beispiel 9 nach Aushärtung und Konditinierung der Klebung gemessen. Die Messung bei Raumtemperatur ergab eine Zugfestigkeit von 5,1 N/mm², die Zugfestigkeit nach Auslagerung in Wasser bei einer Temperatur oberhalb von 95°C während 16 Stunden eine Zugfestigkeit von 6,1 N/mm², wiederum bei Raumtemperatur gemessen.

### Beispiel 11: Zugfestigkeitsprobe auf silberbeschichteter Keramik

Eine silberbeschichtete Keramik mit 10 mm wurde mit 5-(p-Vinylbenzylazo)-8-hydroxychinolin, wie in Beispiel 10 dargestellt, beschichtet. Nach Aushärtung und Konditionierung der Klebung mit Polyurethan-Gießharz wurde die Zugfestigkeit wie in Beispiel 9 gemessen. Bei Raumtemperatur ergab sich ein Festigkeitswert von 4 N/mm², nach einer Auslagerung in Wasser bei einer Temperatur oberhalb 95°C über 16 Stunden ein Festigkeitswert von 1,6 N/mm², wiederum bei Raumtemperatur gemessen.

### Beispiel 12: Zugfestigkeitsprobe auf silberbeschichteter Keramik

Eine silberbeschichtete Keramik mit 10 mm wurde, wie in Beispiel 10 dargestellt mit 5-(p-Aminobenzylazo)-8-hydroxychinolin beschichtet. Die Zugfestigkeit des Polyurethan-Gießharzes wurde nach Aushärtung und Konditionierung der Klebung gemessen, wie in Beispiel 9 dargestellt. Die Zugfestigkeitsprobe bei Raumtemperatur ergab einen Wert von 5,2 N/mm²; nach Auslagerung in Wasser bei einer Temperatur oberhalb 95°C während 16 Stunden ein Wert von 2,2 N/mm², wiederum bei Raumtemperatur gemessen.

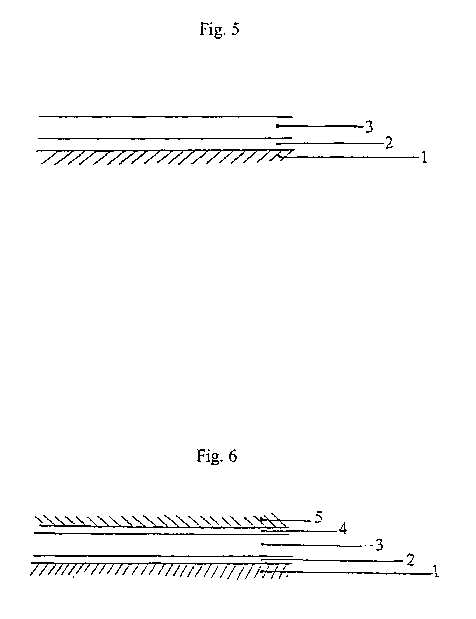

Die erfindungsgemäßen Haftvermittler können in sehr geringer Schichtdicke, d.h. ggf. in monomolekularer Schicht auf die metallische Oberfläche aufgetragen werden. Die aufeinanderfolgenden Schichten, nämlich der erfindungsgemäße Haftvermittler direkt auf eine metallischen Oberfläche, eine mit der reaktiven polymerspezifischen Gruppe des Haftvermittlers reagierende Polymerschicht kann beim Aufbau eines elektronischen Bauelementes wiederum mit einer Schicht des erfindungsgemäßen Haftvermittlers, der auf eine metallische Oberfläche geschichtet ist, beschichtet werden. Insgesamt ergibt sich der in den Figuren 5 und 6 dargestellte Aufbau.

Dabei ist in Figur 5 die Schicht 1 eine metallische Oberfläche, Schicht 2 die Haftvermittlerschicht und Schicht 3 eine organische Polymerschicht.

In Figur 6 ist Schicht 1 eine metallische Schicht, Schicht 2 die Haftvermittlerschicht, Schicht 3 eine organische Polymerschicht, Schicht 4 eine weitere Haftvermittlerschicht und Schicht 5 eine weitere metallische Schicht.

Die Schichten 1 in Figuren 5 und 6 können auch eine dünne Metallschicht sein, die auf eine Unterlage aufgebracht wurde, wie z.B. eine Gold- oder Silberbeschichtung auf einer Piezokeramik.

Figur 6 zeigt einen Aufbau, bei dem zwei gleiche oder unterschiedliche metallische Oberflächen, die Schichten 1 bzw. 5, mit jeweils einer Schicht des Haftvermittlers bedeckt sind, die an gegenüberliegenden Seiten an eine gemeinsame organische Polymerschicht angrenzen. Die Haftvermittlerschichten 2 und 4 können dabei aus dem gleichen 8-Hydroxychinolinderivat bestehen, oder aus verschiedenen, solange sie nur reaktive Gruppen tragen, die mit monooder oligomeren der organischen Polymerschicht 3 reagieren können. Die organische Polymerenschicht 3 kann hier ein Gemisch verschiedener Monomere sein, z.B. Monomeren, die Polyesther, Polyurethane, Polycarbonate und/oder Polyamide bilden können.

Die in der vorangehenden Beschreibung, in der Zeichnung sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Verwendung eines 8-Hydroxychinolinderivats als Haftvermittler zwischen einer metallischen Oberfläche und einer organischen Polymerschicht, das mindestens eine zur Polymerisation fähige Gruppe trägt,
**dadurch gekennzeichnet, daß** das 8-Hydroxychinolinderivat durch Azokupplung von 8-Hydroxychinolin mit organischen Diazoniumverbindungen erhältlich ist, und die allgemeine Formel hat: 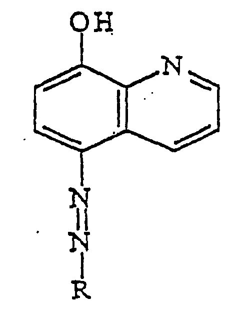 wobei R die mindestens eine zu Polymerisation mit Komponenten organischer Polymere fähige Gruppe trägt.

2. Verwendung nach Anspruch 1, **dadurch gekennzeichnet, daß** die mindestens eine zur Polymerisation mit Komponenten organischer Polymere fähige Gruppe eine freie Amino-, Carboxy-, Epoxy-, Hydroxy- oder Alkenylgruppe ist.

3. Verwendung nach Anspruch 1, **dadurch gekennzeichnet, daß** das 8-Hydroxychinolinderivat eine Struktur hat gemäß:

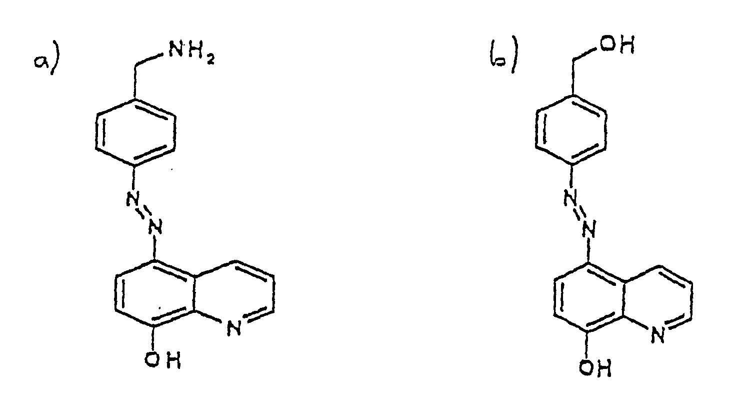

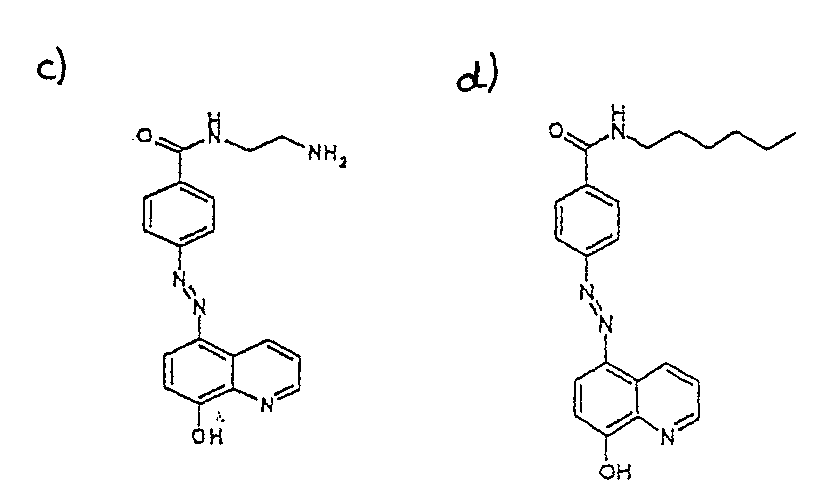

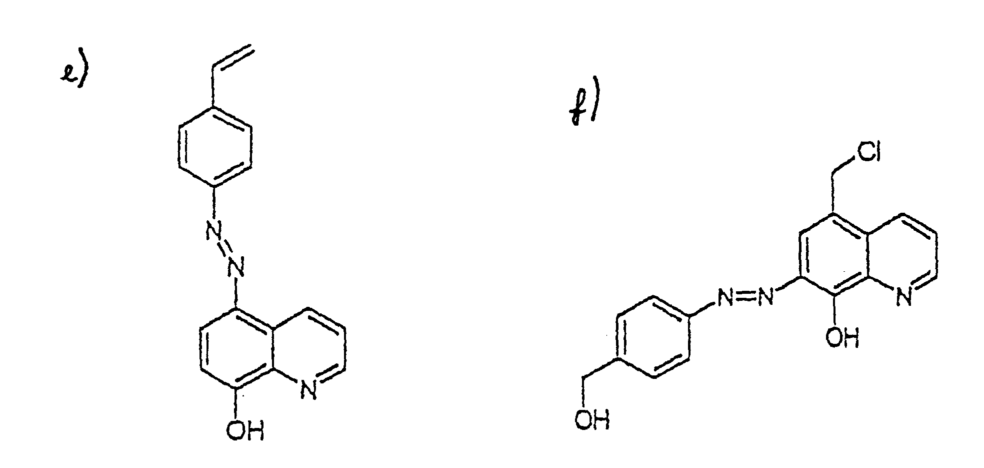

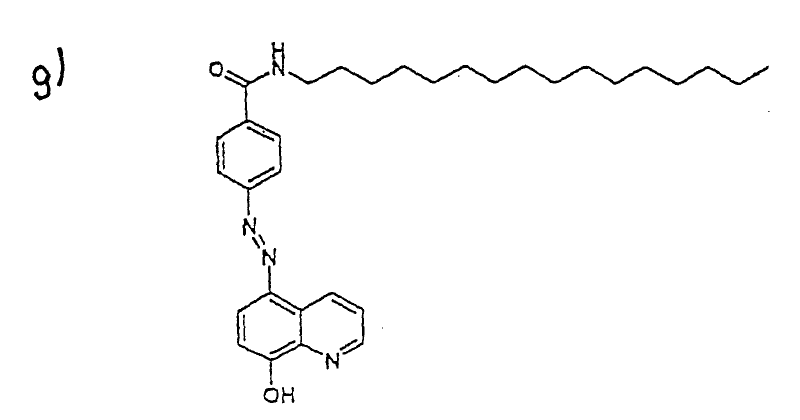

4. Verwendung nach einem der voranstehenden Ansprüche, **dadurch gekennzeichnet, daß** eine Mischung von 8-Hydroxychinolinderivaten verwendet wird.

5. Metallische Oberfläche, die durch Verwendung nach einem der voranstehenden Ansprüche mit Haftvermittler beschichtet ist.

6. Metallische Oberfläche nach Anspruch 5, bei der der Haftvermittler mit Komponenten organischer Polymere polymerisiert ist.

7. Metallische Oberfläche nach Anspruch 5 oder 6, bei der die metallische Oberfläche eine Eisenlegierung oder eine mit Silber oder Gold beschichtete Keramikoberfläche ist.

## Claims

1. Use of an 8-hydroxyquinoline derivative as a bonding agent between a metal surface and an organic polymer layer bearing at least one group capable of polymerisation,
**characterised in that** the 8-hydroxyquinoline derivative is obtainable by azo coupling of 8-hydroxyquinoline with organic diazonium compounds and has the general formula: 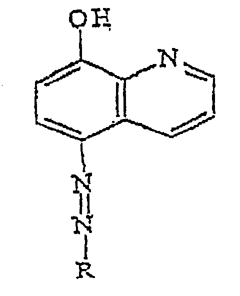 wherein R bears the at least one group capable of polymerisation with components of organic polymers.

2. Use according to claim 1, **characterised in that** the at least one group capable of polymerisation with components of organic polymers is a free amino, carboxy, epoxy, hydroxy or alkenyl group.

3. Use according to claim 1, **characterised in that** the 8-hydroxyquinoline derivative has a structure as follows:

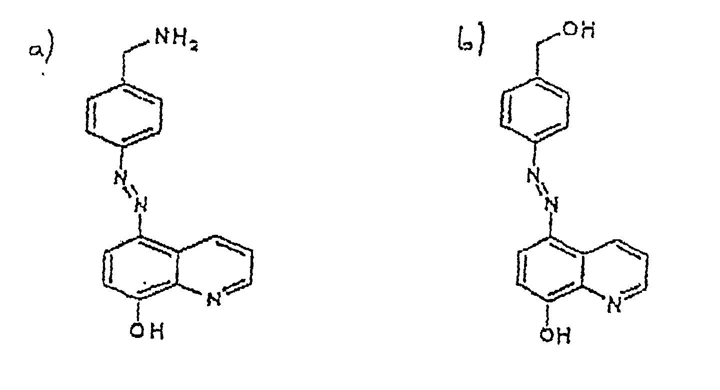

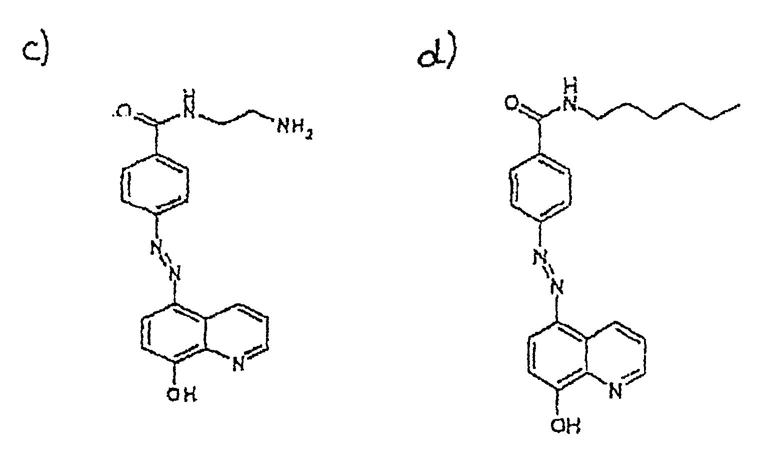

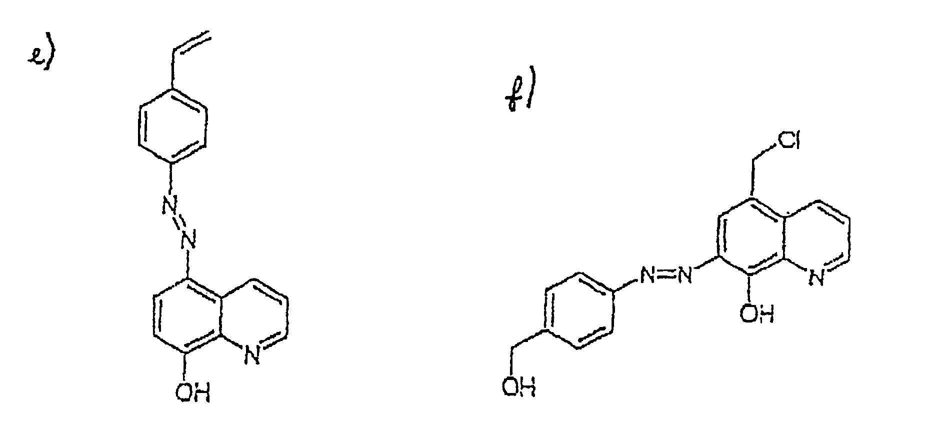

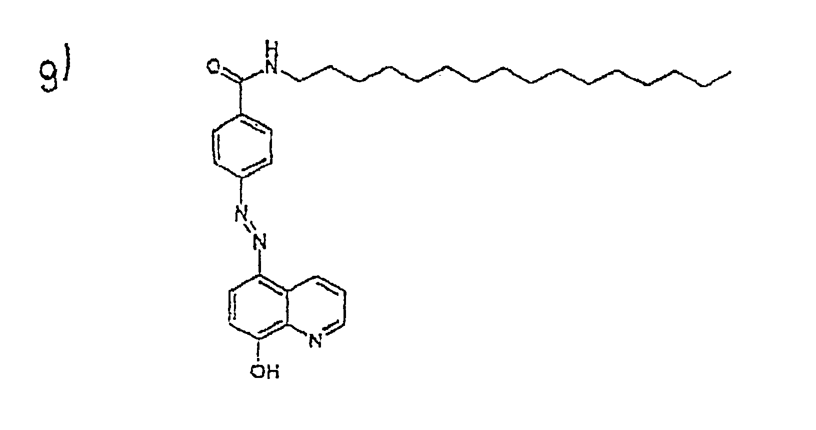

4. Use according to any of the preceding claims,
**characterised in that** a mixture of 8-hydroxyquinoline derivatives is used.

5. A metal surface coated with bonding agent by use according to any of the preceding claims.

6. A metal surface according to claim 5, wherein the bonding agent is polymerised with components of organic polymers.

7. A metal surface according to claim 5 or 6, wherein the metal surface is an iron alloy or a ceramic surface coated with silver or gold.

## Revendications

1. Utilisation d'un dérivé de 8-hydroxyquinoléine en tant qu'agent adhésif entre une surface métallique et une couche polymère organique, qui porte au moins un groupe apte à la polymérisation,
**caractérisée en ce que** le dérivé de 8-hydroxyquinoléine peut être obtenu par copulation azoïque de la 8-hydroxyquinoléine avec des composés diazonium organiques, et qui possède la formule générale : 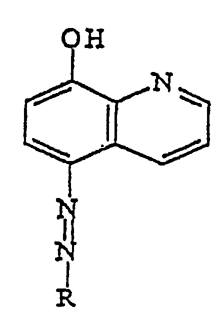 dans laquelle R porte ledit au moins un groupe apte à la polymérisation avec des composants de polymères organiques.

2. Utilisation selon la revendication 1, **caractérisée en ce que** ledit au moins un groupe apte à la polymérisation avec des composants de polymères organiques, est un groupe amino, carboxy, époxy, hydroxy ou alcényle, libre.

3. Utilisation selon la revendication 1, **caractérisée en ce que** le dérivé de 8-hydroxyquinoléine présente une possède une structure conformément à :

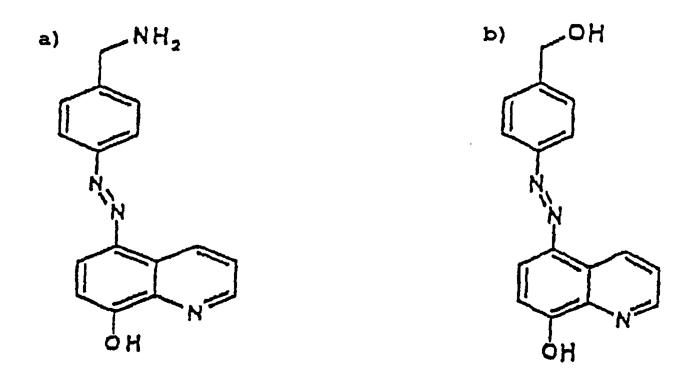

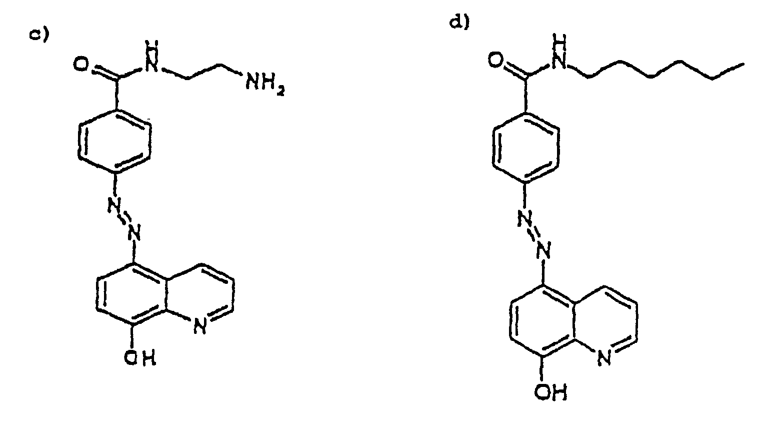

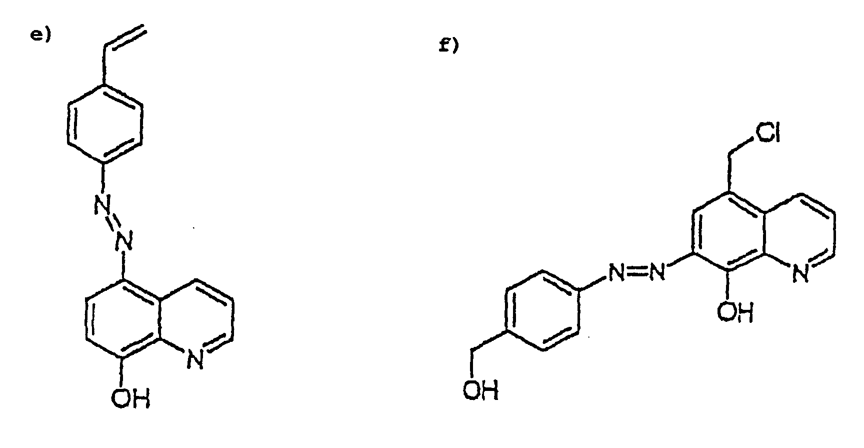

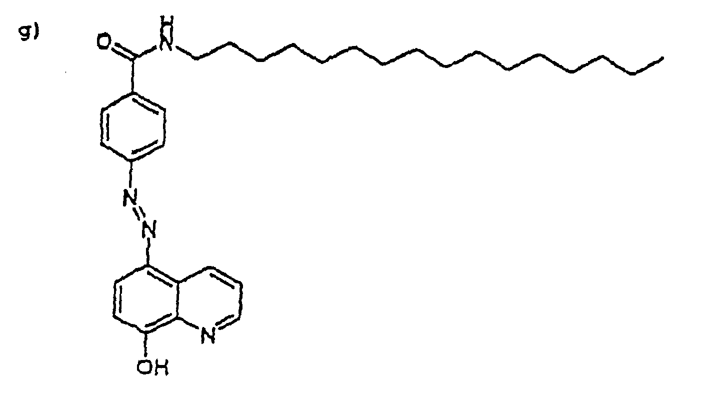

4. Utilisation selon l'une quelconque des revendications précédentes, **caractérisée en ce qu'**un mélange de dérivés de 8-hydroxyquinoléine est employé.

5. Surface métallique qui est revêtue d'un agent adhésif à l'aide d'une utilisation selon l'une quelconque des revendications précédentes.

6. Surface métallique selon la revendication 5, dans laquelle l'agent adhésif est polymérisé avec des composants de polymères organiques.

7. Surface métallique selon la revendication 5 ou 6, dans laquelle la surface métallique est un alliage de fer ou une surface céramique revêtue d'argent ou d'or.
